# EUROPEAN PATENT APPLICATION

(11) **EP 1 726 646 A1**
(43) Date of publication of application: **29.11.2006**
(21) Application number: 05719700.6
(22) Date of filing: 01.03.2005
(51) Int. Cl.: C12N 15/09, C12Q 1/68, G01N 33/574, G01N 33/576

(54) **METHOD OF DETECTING CARCINOGENESIS CAUSED BY HEPATITIS B VIRUS**

(30) Priority: 05.03.2004 JP 2004063046
(71) Applicant: OTSUKA PHARMACEUTICAL CO., LTD., Chiyoda-ku Tokyo 101-8535 (JP)
(72) Inventor: INOUE, Ituro, c/o The Institute of Medical Science, Minato-ku, Tokyo 1088639 (JP); SAIGO, Kenichi, Chuo-ku, Chiba-shi, Chiba 2600026 (JP)
(74) Representative: HOFFMANN EITLE
(86) International application number: PCT/JP2005/003383
(87) International publication number: WO 2005/085436

(57) **Abstract**

A method for detecting, in liver tissue isolated from a human subject and suspected of being cancerous, integration of HBV-DNA into the MLL4 gene, this integration of HBV-DNA indicating the cancerous nature of a tissue. In a typical case, this integration can be detected by carrying out PCR using a primer specific to the region containing intron 3 of the MLL4 gene and a primer specific to the X gene region of HBV.

## Description

### TECHNICAL FIELD

The present invention relates to a method and kit for detecting the integration of HBV-DNA into the MLL4 gene, which indicates that tissue from diseased tissue such as, for example, chronic hepatitis or liver cirrhosis, is cancerous, or to a method and kit for detecting fusion products thereof. The present invention further relates to a method and kit for detecting chromosomal translocation between intron 3 of the MLL4 gene on the long arm of chromosome 19 and the short arm of chromosome 17, which indicates that a tissue is cancerous.

### BACKGROUND ART

The human hepatitis B virus (HBV), which is in the hepadnavirus family, exhibits a very restricted host range and shows a strong tropism for liver parenchymal cells. Acute HBV infection results in serious illness in some cases, and about 0.5% of patients die of fulminant hepatitis. Chronic infection, on the other hand, causes moderate to severe liver diseases, such as, for example, chronic hepatitis, liver cirrhosis, and hepatocellular carcinoma (Beasley, R.P., et al., Lancet, 2, 1129-1133 (1981); Block, T.M., et al., Oncogene, 22, 5093-5107 (2003)). About 25% of patients with a chronic HBV infection ultimately conclude with untreatable liver cancer.

HBV frequently integrates into the human host genome, and the insertional mutagenesis caused by this is presumed to play a major role in oncogenesis. As a consequence, the identification of viral integration sites could provide an effective tool for the identification of cancer-related genes (Gozuacik, D., et al., Oncogene, 20, 6233-6240 (2001); Brechot, C., Semin. Cancer Biol., 10, 211-231 (2000)). The following facts support this.

For example, in the woodchuck animal model, the HBV-related woodchuck hepatitis virus (WHV) integrates at a frequency of at least 80% within or in a region adjacent to the N-myc family oncogenes that can cause oncogenesis in the woodchuck liver (Hsu, T., et al., Cell, 55, 627-635 (1988); Fourel, G., et al., Nature, 347, 294-298 (1990)). In humans, the random integration of HBV-DNA into the host genome has been found in chronic hepatitis tissue (Takada, S., et al., J. Virol., 64, 822-828 (1990)). Furthermore, HBV-DNA clonal or oligoclonal integration has been confirmed by Southern blotting analysis in the hepatocellular carcinoma (HCC) tissue of about 90% of HBs antigen-positive patients (Brechot, C., Semin. Cancer Biol., 10, 211-231 (2000)).

Hepatocellular carcinoma is one of the most common cancers in the world. Hepatocellular carcinoma is produced in the great majority of cases by chronic infection with the human hepatitis B virus or the human hepatitis C virus (HCV). The classic mechanism of oncogenesis by tumor-related viruses proceeds through integration of the viral genome into the cellular genome, thereby inducing activation of a cellular gene that can potentially evoke transformation.

For example, HBV genome integration into the retinoic acid receptor β gene and cyclin A2 gene has been shown to cause oncogenesis (Dejean, A., et al., Nature, 322, 70-72 (1986); Wang, J., et al., Nature, 343, 555-557 (1990)). It has been reported that sarco/endoplasmic reticulum calcium ATPase (SERCA) is also a target for HBV integration and that an HBx/SERCA1 chimeric protein fusion product may be related to oncogenesis via apoptosis (Chami, M., et al., Oncogene, 19, 2877-2886 (2000); Minami, M., et al.). However, oncogenesis by HBV integration at these sites has not been reproduced in samples from other hepatocellular carcinoma patients.

There are also several reports that HBV-DNA preferentially integrates into the promoter region of the human telomerase reverse transcriptase (hTERT) gene in hepatocellular carcinoma tissue, resulting in cis-activation of human telomerase reverse transcriptase in hepatocellular carcinoma (Ferber, M.J., et al., Oncogene, 22, 3813-3820 (2003); Paterlini-Brechot, P., et al., Oncogene, 22, 3911-3916 (2003)). In addition, tumor suppressor genes and oncogenes have been reported to be related to suppression of carcinogenesis by hTERT (Lin, S.Y., and Elledge, S.J., Cell, 113, 881-889 (2003)). Based on these reports, it can be concluded that integration of HBV-DNA into the hTERT gene or its promoter may have a direct effect on oncogenesis.

The mixed lineage leukemia 4 (MLL4) gene (ATCC accession number: AD000671) was reported as the second human homolog to the MLL gene (Ruault, M., et al., Gene, 284, 73-81 (2002)). The MLL4 gene was previously called MLL2, and labeled with MLL2 in the article. This MLL4 gene is a member of the TRX/MLL gene family (FitzGerald, K.T., and Diaz, M.O., Genomics, 59, 187-192 (1999)). The MLL4 gene is located at chromosome 19q13.1 at which frequent genomic rearrangement or amplification has been reported for this gene in solid tumors (Mitelman, F., et al., Nat. Genet., 15, 417-474 (1997); Curtis, L.J., et al., Genomics, 53, 42-55 (1998); Ferbus, D., et al., Int. J. Cancer, 80, 369-372 (1999)). This gene is ubiquitously expressed in adult human tissue. Gene amplification at chromosome 19q13.1 in HBV-related hepatocellular carcinoma has been observed by comparative genomic hybridization (CGH) (Marchio, a., et al., Genes Chromosom. Cancer, 18, 59-65 (1997)). High-frequency genomic rearrangement of chromosome 19q13.1 has also been reported in solid tumors (Curtis, L.J., et al., Genomics, 53, 42-55 (1998); Ferbus, D., et al., Int. J. Cancer, 80, 369-372 (1999); Urashima, T., et al., J. Hepatol., 26, 771-778 (1997)).

It is known that the MLL gene is a frequent target of chromosomal translocations related to human leukemia (der Poel, S.Z., et al., Proc. Natl. Acad. Sci. U.S.A., 88, 10735-10739 (1991); Rowley, J.D., Annual Rev. Genet., 96, 496-519 (1998); Huntsman, D.G., et al., Oncogene, 18, 7975-7984 (1999)). Due to the existence of highly conserved regions between MLL and MLL4, the analogous possibility could also be true for the MLL4 gene, but there have been no reports that the chromosomal region containing the MLL4 gene is involved in translocation in leukemia.

Notwithstanding the thinking that HBV-DNA is connected to oncogenesis in human liver cells, there is scarce information on liver cancer-inducing sites of HBV-DNA integration into human chromosomes, and for this reason HBV-originating liver cancer cannot be detected at high probabilities. Given this, there is demand for the identification of HBV-DNA integration sites in human chromosomes in HBV-originating liver cancer.

### DISCLOSURE OF THE INVENTION

An object of the present invention is to provide a method and a kit using same, that can effectively detect, for example, liver cancer by identifying, in liver tissue suspected of being cancerous, a site of HBV-DNA integration into the MLL4 gene and translocation between chromosomes 17 and 19, thereby indicating that the tissue is cancerous.

In order to achieve this object, the present inventors pursued the identification of novel target genes for the preferential and cancer-indicating integration of HBV-DNA. In addition, the present inventors carried out extensive investigations for the purpose of establishing a methodology that, by detecting integration into a specific gene or by detecting fusion products that could be produced as a result, would detect that the sample provided by a patient is liver cancer linked to the abnormal functioning of the specific gene or has the potential to become liver cancer in the future. The following knowledge was acquired as a result of these investigations.
(1) DNA was extracted from liver tumor tissue from 10 hepatocellular carcinoma patients that were HBs antigen positive and was subjected to adaptor-ligation/suppression PCR (Siebert, P.D., et al., Nucleic Acids Res., 23, 1087-1088 (1995)) using HBV-DNA-specific primers. Adaptor-ligation/suppression PCR is a method that can amplify a specific base sequence corresponding to the primer along with the flanking site where this specific base sequence is inserted. This procedure enables identification of an HBV-DNA integration site in DNA extracted from tumor tissue.
   As a result, HBV-DNA integration was detected in 7 of 10 hepatocellular carcinoma samples, and HBV-DNA integration in intron 3 of the MLL4 gene was confirmed in 4 of these samples.
   These observations support the idea that liver cancer caused by HBV infection can be detected by detecting the integration of HBV-DNA in intron 3 of the MLL4 gene.
(2) HBV promoter region and coding region for HBV X protein excluding the C-terminus were present in the DNA from the 4 samples confirmed to have HBV-DNA integration in intron 3 of the MLL4 gene. This suggests the possible presence of fusion transcription products between MLL4 and the X gene of HBV (HBx). According to the actual results from RT-PCR, fusion transcription products were produced in which the reading frame was shifted by the insertion of HBx-DNA and a premature stop codon was thereby produced in the MLL4 gene; fusion transcription products were also produced in which the reading frame was not shifted and a premature stop codon was not introduced in the MLL4 gene.
   These observations support the idea that liver cancer caused by HBV infection can be detected by detecting HBx-DNA/MLL4 gene fusion transcription products.
(3) The total protein was prepared from the liver tumor tissue and from adjacent nontumorous tissue for the 4 samples in which HBx-DNA integration into intron 3 of MLL4 had been confirmed, and detection of immunoprecipitation products and Western blotting using anti-HBx monoclonal antibody were carried out. This resulted in the detection of bands or immunoprecipitation products that were observed in the tumorous tissue but not in the nontumorous tissue.
   These observations support the idea that liver cancer caused by HBV infection can be detected by detecting HBV-MLL4 fusion protein using Western blotting or immunoprecipitation reactions using anti-HBx monoclonal antibody or monoclonal antibody against fusion protein from intron 3 of MLL4 and individual regions of HBV X.
(4) PCR was carried out using primer corresponding to intron 3 of MLL4 (long arm of chromosome 19) and primer corresponding to the short arm of chromosome 17, on 26 samples from hepatocellular carcinoma tissue samples that were anti-HBc antibody positive, including 10 samples that were HBs antigen positive. Chromosomal translocation between intron 3 of MLL4 (long arm of chromosome 19) and the short arm of chromosome 17 was detected in 22 samples.
   This observation supports the idea that liver cancer caused by HBV infection can be detected at high probabilities by detecting, using PCR, chromosomal translocation between intron 3 of MLL4 (long arm of chromosome 19) and the short arm of chromosome 17.
(5) The preceding results strongly suggest that intron 3 of MLL4 is a preferential target for oncogenesis-linked HBV integration. In addition, chromosomal rearrangement in this region may also be etiologically linked with oncogenesis in the liver.

The present invention was completed based on the findings described above and provides detection methods and detection kits as described below.
1. A method for detecting, in liver tissue isolated from a human subject and suspected of being cancerous, integration of HBV-DNA into the MLL4 gene, this integration of HBV-DNA indicating the cancerous nature of a tissue.
2. The method according to item 1, for detecting integration of HBV-DNA into the MLL4 gene by
   conducting a polymerase chain reaction (PCR) on DNA from the liver tissue; and
   confirming the integration of HBV-DNA into the MLL4 gene by determining the base sequence of the amplified DNA.
3. The method according to item 2, wherein the integration of a region containing the HBV-DNA X gene into intron 3 of the MLL4 gene is confirmed.
4. The method according to item 1, for detecting the integration of HBV-DNA into intron 3 of the MLL4 gene.
5. The method according to item 4, for detecting the integration of HBV-DNA into the region of intron 3 of the MLL4 gene from base number 17515 to 17818 from the 5' end.
6. The method according to item 1, 4, or 5, wherein the HBV-DNA is a region containing the X gene of HBV.
7. A method for detecting, in liver tissue isolated from a human subject and suspected of being cancerous, integration of HBV-DNA into the MLL4 gene, this integration of HBV-DNA indicating the cancerous nature of a tissue, by a procedure comprising the steps of
   (1) extracting DNA from the liver tissue;
   (2) carrying out PCR using, with the DNA obtained in (1) being used as template, a first primer specific to the region containing intron 3 of the MLL4 gene and a first primer specific to the X gene region of HBV; and
   (3) optionally carrying out PCR again using, with the DNA amplified in (2) being used as template, a second primer specific to the region containing intron 3 of the MLL4 gene and a second primer specific to the X gene region of HBV.
8. The method according to any of items 1, 4, 5, and 6, wherein integration of HBV-DNA into the MLL4 gene is detected by detecting an MLL4 gene/HBV fusion transcription product.
9. The method according to item 8, for detecting an MLL4 gene/HBV X region fusion transcription product.
10. The method according to any of items 1, 4, 5, and 6, wherein integration of HBV-DNA into the MLL4 gene is detected by detecting an MLL4/HBV fusion protein.
11. The method according to item 10, comprising detecting an MLL4/HBV X region fusion protein.
12. The method according to item 10 or 11, which uses an antibody or antibody fragment that specifically binds to an MLL4/HBV X region fusion protein.
13. A method for detecting, in liver tissue isolated from a human subject and suspected of being cancerous, a t(17;19)(p11.2;q13.1) chromosomal translocation of the MLL4 gene, this translocation indicating the cancerous nature of a tissue.
14. The method according to item 13, for detecting the t(17;19) (p11.2;q13.1) chromosomal translocation of the MLL4 gene by detecting a base sequence that includes a junction between chromosome 17 and chromosome 19.
15. The method according to item 14, for detecting a base sequence containing a junction between chromosome 17 and chromosome 19 by a procedure comprising the steps of:
   (1) extracting DNA from the liver tissue;
   (2) carrying out PCR using, with the obtained DNA being used as template, a first primer specific to the region containing p11.2 of chromosome 17 and a first primer specific to the region containing intron 3 of the MLL4 gene at q13.1 of chromosome 19; and
   (3) carrying out PCR using, with the amplified DNA being used as template, a second primer specific to the region containing p11.2 of chromosome 17 and a second primer specific to the region containing intron 3 of the MLL4 gene at q13.1 of chromosome 19.
16. A kit for detecting the integration of HBV-DNA into the MLL4 gene.
17. A kit for detecting the integration of HBV-DNA into intron 3 of the MLL4 gene.
18. A kit for detecting the integration of the X region of HBV into intron 3 of the MLL4 gene.
19. The kit according to item 18, comprising a primer or probe specific to the region containing intron 3 of the MLL4 gene and a primer or probe specific to the X region of HBV.
20. A kit for detecting an MLL4 gene/HBV X region fusion transcription product.
21. A kit for detecting an MLL4 gene/HBV X region fusion protein.
22. The kit according to item 21, comprising an antibody or antibody fragment that specifically binds to an MLL4/HBV X region fusion protein.
23. A kit for detecting a t(17;19)(p11.2;q13.1) chromosomal translocation of the MLL4 gene.
24. The kit according to item 23, comprising a primer or probe specific to the region containing intron 3 of the MLL4 gene and a primer or probe specific to the region containing p11.2 of chromosome 17.
25. The method according to item 7, that uses the primer shown by SEQ ID NO. 12 as the primer specific to the region containing intron 3 of the MLL4 gene and that uses the primer shown by SEQ ID NO. 4 as the primer specific to the X gene region of HBV.
26. The method according to item 7, that uses the primer shown by SEQ ID NO. 8 as the first primer specific to the region containing intron 3 of the MLL4 gene and that uses the primer shown by SEQ ID NO. 14 as the first primer specific to the X gene region of HBV, and optionally that uses the primer shown by SEQ ID NO. 9 as the second primer specific to the region containing intron 3 of the MLL4 gene and that uses the primer shown by SEQ ID NO. 14 as the second primer specific to the X gene region of HBV.
27. The method according to item 15 that uses the primer with SEQ ID NO. 20 as the first primer specific to the region containing p11.2 of chromosome 17, the primer with SEQ ID NO. 8 as the first primer specific to the region containing intron 3 of the MLL4 gene at q13.1 of chromosome 19, the primer with SEQ ID NO. 21 as the second primer specific to the region containing p11.2 of chromosome 17, and the primer with SEQ ID NO. 9 as the second primer specific to the region containing intron 3 of the MLL4 gene at q13.1 of chromosome 19.

With regard to the abovementioned DNA, the methods and kits according to the present invention enable the detection by PCR of the integration of HBV-DNA into the MLL4 gene in DNA from liver tissue collected from a human for whom liver cancer is suspected, and particularly a human with HBV infection and/or chronic hepatitis or liver cirrhosis, and thereby enable detection of the presence/absence of neoplastic change in hepatocyte tissue based on an indication thereof.

In addition, the methods and kits according to the present invention can detect a fusion product yielded by the integration of HBV-DNA into the MLL4 gene in the aforementioned DNA and more particularly can detect a fusion product, fusion transcription product, or fusion protein yielded by the integration of DNA containing the X gene region of HBV into the MLL4 gene, thereby enabling genetic diagnosis with respect to tumorigenesis. Moreover, these fusion products can be used in research into the linkage between carcinogenesis and gene integration in patients with liver diseases such as chronic hepatitis and liver cirrhosis.

The methods and kits according to the present invention can also detect a t(17;19)(p11.2;q13.1) chromosomal translocation of the MLL4 gene, which again enables genetic diagnosis with respect to tumorigenesis. In addition, the methods and kits according to the present invention can be used in research into the relationship between cell transformation, and particularly hepatocyte transformation, and the function of genes linked to genetic rearrangement.

In view of the preceding, the methods and kits according to the present invention can provide information and measures useful for the elucidation of the mechanism of onset of oncogenesis in liver tissue and particularly for the elucidation of the mechanism of onset of hepatocellular carcinoma, and for understanding, diagnosing, and preventing oncogenesis in tissues, with respect to various diseases, for example, for humans suffering from chronic hepatitis or liver cirrhosis, alcoholic hepatopathy, hepatopathy based on a metabolic disease such as Wilson's disease, hemochromatosis, hepatopathy based on exposure to a chemical, such as aflatoxin, or autoimmune hepatitis.

### BRIEF DESCRIPTION OF THE DRAWINGS

Figure 1 shows the results of specific PCR and adaptor-ligation/suppression PCR for detecting HBV integration sites.
Figure 1a shows the protocol for adaptor-ligation/suppression PCR.
Figure 1b shows the results of screening for HBV/cellular DNA junctions by adaptor-ligation/suppression PCR.
Figure 1c shows the PCR conformation with specific primers for HBV-DNA integration into the MLL4 gene.
Figure 2 shows the sequences flanking HBV integration sites.
Figure 3 shows the results of analysis of four integrated HBV genomes.
Figure 4 shows the results of RT-PCR analysis of HBx-MLL4 fusion transcription products.
Figure 5 shows the immunodetection results for HBx fusion proteins in samples from hepatocellular carcinoma patients.
Figure 6 shows reciprocal translocation at the gene locus for intron 3 of MLL4.

### BEST MODE FOR CARRYING OUT THE INVENTION

The present invention is described in detail in the following.

The present invention has been achieved based on the discovery of the following facts: an indication of cancerous tissue in hepatocellular carcinoma cases presenting a history of chronic hepatitis or liver cirrhosis is connected to HBV-DNA integration into a specific region of the MLL4 gene and particularly integration of the X gene region of HBV into the MLL4 gene; an indication of cancerous tissue in hepatocellular carcinoma cases presenting a history of chronic hepatitis or liver cirrhosis is connected to translocation between chromosome 17 and the MLL4 gene residing on chromosome 19; and, based on these facts, the risk of onset of, for example, hepatocellular carcinoma, can be determined (predictive diagnosis) through detection of DNA integration or gene translocation at a specific location in the MLL4 gene.

The abbreviations used in this Description for amino acids, peptides, base sequences, nucleic acids, and so forth follow the IUPAC-IUB rules (IUPAC-IUB Communication on Biological Nomenclature, Eur. J. Biochem., 138:9 (1984)), "Guidelines for Preparing Descriptions Containing Base Sequences or Amino Acid Sequences" (edited by the Japan Patent Office), and the symbols customary in the concerned field.

### (I) The method of detecting HBV-DNA integration into the MLL4 gene

A first detection method according to the present invention is a method that detects, in liver tissue isolated from a human subject and suspected of being cancerous, integration of HBV-DNA into the MLL4 gene, this integration indicating the cancerous nature of a tissue.

### Target population

There are no particular limitations on the human population that is the target for the first examination method according to the present invention, and this first examination method may be applied to normal healthy individuals and to humans suffering from a disease such as a liver disease. Among the preceding, this first examination method is very suitably targeted to humans suffering from chronic hepatitis, liver cirrhosis, alcoholic hepatopathy, hepatopathy based on a metabolic disease such as Wilson's disease, hemochromatosis, hepatopathy caused by exposure to a chemical such as aflatoxin, or autoimmune hepatitis, which are diseases that readily progress to hepatocellular carcinoma. The examination method according to the present invention can also be used to confirm liver cancer caused by HBV infection in humans suffering from liver cancer.

### The MLL4 gene

The genomic sequence of the human MLL4 gene given in this Description conforms to the genomic sequence of the gene with a total base length (bp) of 46,251 reported under GenBank accession number AD000671 by J. E. Lamerdin et al., of the Human Genome Center. SEQ ID NO. 1 shows the sequence of bases from exon 3 to exon 4, which is given by the region from base 15295 to base 17983, of the human MLL4 gene (AD000671) with a total base length of 46,251 (bp).

The gene inferred from the genomic sequence for MLL4 comprises 37 exons (FitzGerald, K.T., and Diaz, M.O., Genomics, 59, 187-192 (1999)), and introns are present therebetween. In addition, the gene inferred from the genomic sequence for MLL4 maps to 13.1 of the long arm of chromosome 19 (19q13.1), for which frequent rearrangement and amplification has been reported in solid tumors, and is a human member of the TRX/MLL gene family that is very similar to the MLL gene, which is itself associated with numerous chromosomal translocations and leukemia.

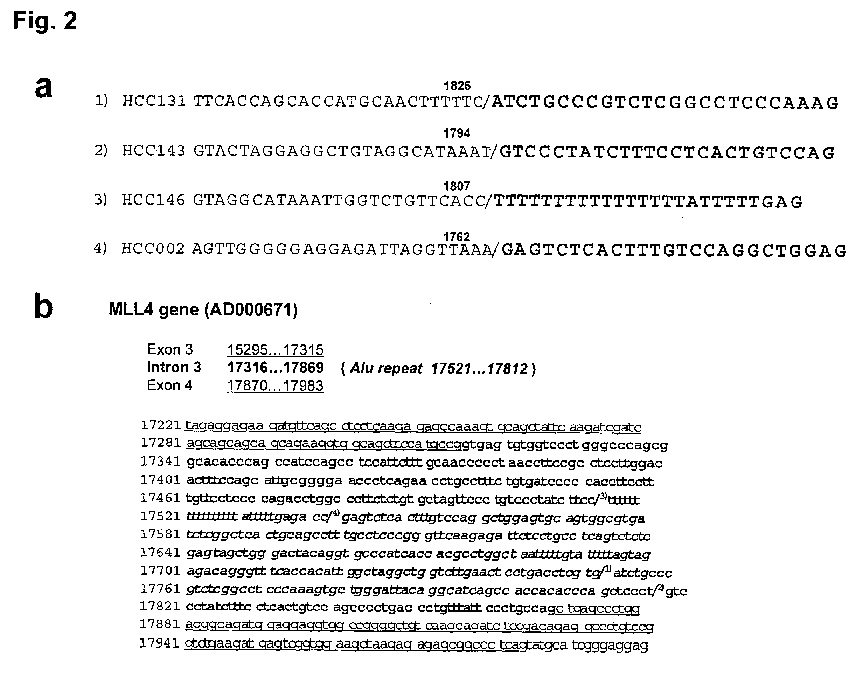

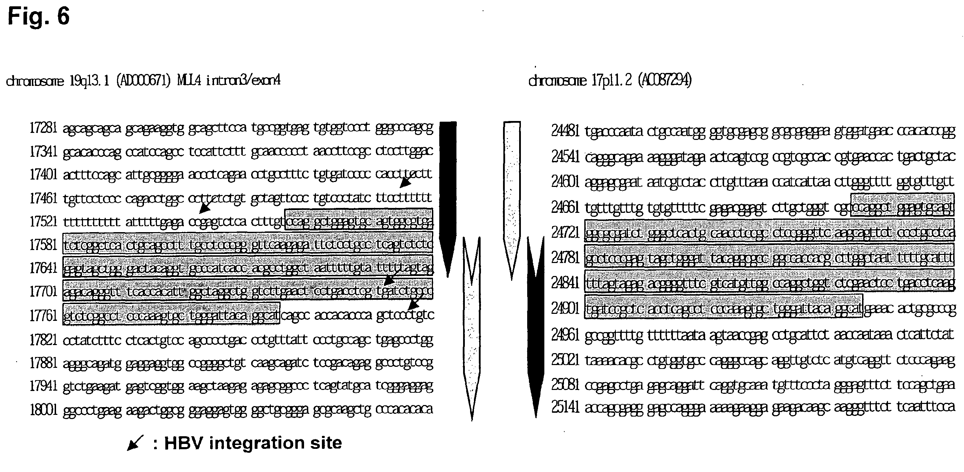

Sequences containing the intron 3/exon 4 region of MLL4 are shown in Figures 2b, 3c, 4b, and 6, and reciprocal translocation between the DNA sequence of the MLL4 gene on chromosome 19 and chromosome 17 is shown in Figure 6 as inset DNA sequences.

### HBV-DNA

The genomic sequence of the human hepatitis B virus (HBV) given in this Description conforms to the base sequence of the gene with a total base length (bp) of 3,215 reported under GenBank accession number AB033550 by Okamoto et al., Jichi Medical School.

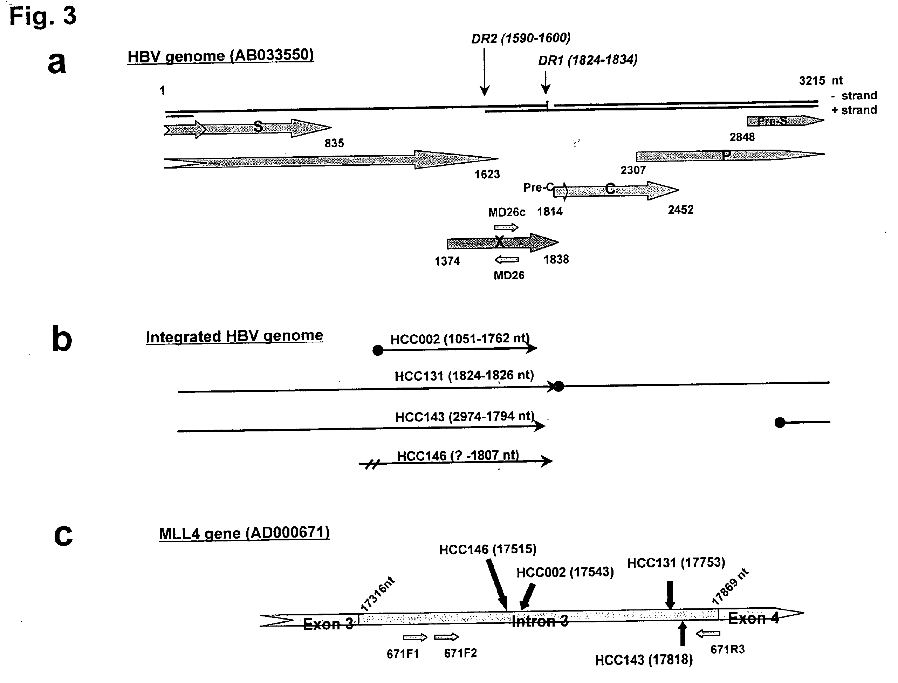

In addition, in the present invention, the X gene region, which is an HBV-DNA region for which integration into the MLL4 gene was confirmed, is the region (SEQ ID NO. 2) with a base length (bp) of 465 given by base numbers 1374 to 1838 under GenBank accession number AB033550. This gene region codes for a protein having an amino acid sequence length of 155. The circular genome of HBV is shown in linear form in Figure 3a.

### Integration of HBV-DNA into the MLL4 gene

The HBV genome that integrated into the chromosomal DNA is shown in Figure 3b for the 4 samples (HCC002(1051-1762), HCC131(1824-1826), HCC143(2974-1794), HCC146(unidentified-1807)) for which HBV-DNA integration was detected in the examples of the present invention. For these 4 samples in which HBV integration was confirmed, full-length HBV integrated only in HCC131, while the X gene promoter region and X gene coding region excluding a C-terminal moiety are integrated in all 4 samples.

Thus, the integration of any portion of HBV-DNA into the MLL4 gene may be detected in the method according to the present invention, but thereamong an examination of the cancerous nature of liver tissue, such as liver cancer, can be effected at even higher probabilities by detecting integration into the MLL4 gene of either the promoter region or the coding region of the X gene of HBV.

As shown in Figure 3c, the position of HBV-DNA integration into the MLL4 gene was within intron 3 in all 4 samples. More specifically, counting from the 5' terminal of the MLL4 gene, the HBV-DNA integrated at position 17515 in sample HCC146, at position 17543 in HCC002, at position 17753 in HCC131, and at position 17818 in HCC143.

A check for hepatocellular carcinoma can therefore be carried out at a high probability in the method according to the present invention through detection of the integration of HBV-DNA into intron 3 of the MLL4 gene. Within this, a check on the cancerous nature of liver tissue, such as liver cancer, can be effected at even higher probabilities by detecting integration of HBV-DNA into the region of the MLL4 gene at 17515 to 17818 from the 5' end.

### DNA preparation

With regard to the extraction of DNA from the human subject, DNA is prepared from liver tissue suspected of being cancerous that has been isolated from the human that is the subject of the examination.

For the purposes of the present invention, "gene" refers not only to double-stranded DNA, but also to the individual single-stranded DNAs, known as the sense strand and the antisense strand, comprising double-stranded DNA; nor is there any limitation whatever with regard to length. Accordingly, and in the absence of specific indications otherwise, a gene (DNA) for the purposes of the present invention includes double-stranded DNA comprising human genomic DNA, single-stranded DNA (sense strand) including cDNA, single-stranded DNA (antisense strand) having a complementary sequence with the sense strand, and fragments of the preceding. A gene (DNA) as referenced by the present invention comprises a regulatory region, coding region, exon, and intron. RNA and DNA are examples of polynucleotides. DNA includes cDNA, genomic DNA, and synthetic DNA, while a polypeptide with a specific amino acid sequence includes fragments, derivatives, homologs, and variants thereof.

The gene used by the examination method according to the present invention can be readily produced or acquired by general genetic engineering methods (refer, for example, to Molecular Cloning, 2nd Ed., Cold Spring Harbor Lab. Press (1989); "Genetic Research Methods I, II, III", Experimental Protocols in Biochemistry Series, edited by The Japanese Biochemical Society (1986)) based on the sequence information in the specific example of the gene disclosed by the present invention.

A specific example is the gene obtained from a suitable source in which the gene is expressed, in the present invention from individuals suffering from chronic hepatitis, liver cirrhosis, alcoholic hepatopathy, hepatopathy based on a metabolic disease such as Wilson's disease, hemochromatosis, hepatopathy caused by exposure to a chemical such as aflatoxin, or autoimmune hepatitis, which are diseases that readily progress to hepatocellular carcinoma. Isolated DNA may preferably be prepared from human tissue from individuals suffering from chronic hepatitis patients, liver cirrhosis patients, or hepatocellular carcinoma patients.

The sample from the aforementioned source material is preferably genomic DNA or DNA of human patient origin. The separation of RNA from these source materials, the separation and purification of mRNA, the acquisition of cDNA and its cloning, and so forth can be carried out by any of the usual methods.

The procedure for screening the genes from the isolated DNA or gene expression products is also not particularly limited, and, for example, the target gene can be recovered by selecting the desired clone using a suitable probe or antibody specific to the gene (for example, Proc. Natl. Acad. Sci., USA, 78, 6613 (1981); Science, 222, 778 (1983)).

Specific examples in this regard are an immunoscreening procedure that uses antibody specific for protein produced by a cDNA to select the corresponding cDNA clone; plaque hybridization using a probe that selectively binds to the target DNA sequence; colony hybridization; and combinations of the preceding.

In addition to the preceding methods, DNA in which HBV-DNA has been integrated into MLL4 can be extracted from source material by adaptor-ligation/suppression PCR. That is, after extraction of genomic DNA from the source material, the genomic DNA is digested with a restriction enzyme to give blunt ends; an adaptor sequence is then ligated to the genomic DNA fragments; and a primer specific to an HBV sequence and an adaptor-specific primer are then added and the target genomic DNA fragment can be amplified in a primary PCR reaction (Siebert, P.D., et al., Nucleic Acids Res., 23, 1087-1088 (1995)). The genomic DNA can be acquired using a Genomewalker kit from Clontech Laboratories, Inc.

The extracted gene can be amplified by gene amplification procedures. Such amplification can make detection in the detection methods according to the present invention easier and more accurate.

A very suitable example of a gene amplification method is DNA/RNA amplification by PCR (Science, 230, 1350 (1985)). Especially in those cases where it is difficult to obtain full-length cDNA from the tissue or from a library, the use of RACE (rapid amplification of cDNA ends, Experimental Medicine, 12(6), 35 (1994)) and particularly 5'-RACE (M.A. Frohman, et al., Proc. Natl. Acad. Sci., USA, 8, 8998 (1988)) is very suitable.

Isolation and purification of the gene fragment amplified by, for example, PCR, can be carried out by ordinary methods, for example, gel electrophoresis or a column method. Confirmation of the amplification product can be carried out by, for example, a mass spectroscopic method. The gene amplified by these procedures is submitted, in accordance with the properties of the amplified material, to detection of whether integration of an HBV-DNA gene into the MLL4 gene according to the present invention is present or determination of the DNA sequence of the integrated fusion product, or its mRNA can be analyzed.

### Detection of integration by PCR

PCR using a primer set for specifically detecting a specific sequence comprising HBV X gene integration into intron 3 of the MLL4 gene is an example of a method that can detect integration of the HBV X gene into intron 3 of the MLL4 gene.

DNA chemically synthesized based on data on the MLL4 gene or HBV-DNA genes can generally be used for the screening primers, but an already acquired fused or bonded gene or fragment thereof can also be favorably used for the screening primer. In addition, gene-specific forward and reverse primers designed based on gene sequence data can be used as the screening primers.

A typical example of PCR using such a primer set is PCR using a primer specific for the region containing intron 3 of the MLL4 gene and a primer specific for the X gene of HBV.

When an amplification product is not obtained at a level enabling detection, the detection sensitivity can be raised by carrying out a secondary PCR using the aforementioned PCR amplification product as template, a primer specific to the region containing intron 3 of the MLL4 gene, and a primer specific to the X gene of HBV.

Specifically, a method comprising the following steps is a typical example of a method that detects, in liver tissue isolated from a human subject and suspected of being cancerous, integration of HBV-DNA into the MLL4 gene, this integration indicating the cancerous nature of a tissue:
(1) extracting DNA from the liver tissue;
(2) carrying out PCR using the DNA obtained in (1) as template, a first primer specific to the region containing intron 3 of the MLL4 gene, and a first primer specific to the X gene region of HBV; and
(3) optionally carrying out PCR again using the DNA amplified in (2) as template, a second primer specific to the region containing intron 3 of the MLL4 gene, and a second primer specific to the X gene region of HBV.

The sequences of the primers (forward and reverse primers) specific to these regions can be appropriately designed according to the usual methods based on the base sequences of the MLL4 gene and HBV-DNA. The length of the primer is not particularly limited, but in general may be at least 10 nucleotides and preferably is about 10 to about 50 nucleotides, more preferably about 10 to about 35 nucleotides, and even more preferably about 15 to 35 nucleotides.

In specific terms, the primer for MLL4 may be a primer that can recognize a partial sequence of about 10 to about 35 nucleotides and preferably about 15 to about 30 nucleotides of intron 3 of MLL4 and can thereby initiate DNA synthesis. For HBV-DNA, the primer may be a primer that can recognize a partial sequence of about 10 to about 35 nucleotides and preferably about 15 to about 30 nucleotides of the HBV X gene sequence inserted in the intron 3 region of MLL4 and can thereby initiate DNA synthesis.

These primers can be synthesized by the usual methods. For example, they can be synthesized by a chemical synthesis method such as the phosphoramidite method or the phosphotriester method, and in addition they can be synthesized using a commercially available automatic oligonucleotide synthesizer, such as the LKB Gene Assembler Plus from Pharmacia. Double-stranded fragments can also be obtained from chemically synthesized single-stranded products, by synthesizing the complementary chains and annealing the two chains under appropriate conditions or by adding the complementary chain using a DNA polymerase together with a suitable primer sequence.

Specific examples of primer pairs are given below. The following combinations, for example, can be used to detect, respectively, the front and back junctions of a region of HBV X gene integration into the region containing intron 3 of the MLL4 gene.

To detect one junction of a region of HBV X gene integration into the region containing intron 3 of the MLL4 gene, the forward primer for the primary PCR reaction can be exemplified by the 671F1 shown by SEQ ID NO. 8 and the reverse primer can be exemplified by the MD26 shown by SEQ ID NO. 14, while the forward primer for the secondary PCR reaction can be exemplified by the 671F2 shown by SEQ ID NO. 9 and the reverse primer can be exemplified by the MD26 shown by SEQ ID NO. 14. This secondary reaction is carried out on an optional basis.

To detect the other junction of a region of HBV X gene integration into the region containing intron 3 of the MLL4 gene, the forward primer for the primary PCR reaction can be exemplified by the MD26c shown by SEQ ID NO. 4, while the reverse primer can be exemplified by the 671R3 shown by SEQ ID NO. 12. 671R1 shown by SEQ ID NO. 10 or 671R2 shown by SEQ ID NO. 11 may also be used as the reverse primer in place of the 671R3 shown by SEQ ID NO. 12.

The following combinations, for example, can be used to detect, respectively, the front and rear junctions in the case of reverse integration of the HBV X gene into the region containing intron 3 of the MLL4 gene.

To detect one junction of a region of HBV X gene reverse integration into the region containing intron 3 of the MLL4 gene, the forward primer for the primary PCR reaction can be exemplified by the 671F1 shown by SEQ ID NO. 8 and the reverse primer can be exemplified by the MD26c shown by SEQ ID NO. 4. In addition, the forward primer for the secondary PCR reaction can be exemplified by the 671F2 shown by SEQ ID NO. 9 and the reverse primer can be exemplified by the MD26c shown by SEQ ID NO. 4. This secondary reaction is carried out on an optional basis.

To detect the other junction of a region of HBV X gene reverse integration into the region containing intron 3 of the MLL4 gene, the forward primer for the primary PCR reaction can be exemplified by the MD26 shown by SEQ ID NO. 14, while the reverse primer can be exemplified by the 671R3 shown by SEQ ID NO. 12. The 671R1 shown by SEQ ID NO. 10 or the 671R2 shown by SEQ ID NO. 11 can be used as the reverse primer instead of 671R3.

Preferred primer pair combinations can be exemplified by the combination of the MD26c shown by SEQ ID NO. 4 as the forward primer and the 671R3 shown by SEQ ID NO. 12 as the reverse primer for junction detection in the case of a region of HBV X gene integration into the region containing intron 3 of the MLL4 gene. For junction detection in the case of HBV X gene reverse integration into the region containing intron 3 of the MLL4 gene, a preferred example is the combination of the 671F1 shown by SEQ ID NO. 8 as the forward primer and the MD26c shown by SEQ ID NO. 4 as the reverse primer.

### Detection and identification of PCR amplification products

The amplification of DNA by the aforementioned PCR can be detected by, for example, agarose gel electrophoresis, thereby enabling detection of HBV X gene integration into intron 3 of the MLL4 gene.

In addition, the integration of HBV-DNA into the MLL4 gene can also be confirmed by extracting this amplification product from a band cut out of the agarose gel and determining its base sequence.

The DNA base sequence determination can be carried out according to the usual methods, for example, the dideoxy method (Proc. Natl. Acad. Sci., USA., 74, 5463 (1977)) or the Maxam-Gilbert method (Methods in Enzymology, 65, 499 (1980)). The base sequence can also be conveniently determined using, for example, a commercial sequencing kit.

### Detection of fusion transcription products

Integration of HBV-DNA into the MLL4 gene can also be detected by detecting MLL4 gene/HBV X gene fusion transcription products. An MLL4 gene/HBV X gene fusion transcription product in the form of the fusion transcription product of all or a portion of MLL4 with all or a portion of the HBV X gene may be detected.

This detection can be done by the usual methods, for example, by measurement at the cellular level using RNA amplification by the reverse transcribed-polymerase chain reaction (RT-PCR, E.S. Kawasaki, et al., Amplification of RNA. In: PCR Protocol, a Guide to Methods and Applications. Academic Press, Inc., San Diego, 21-27 (1991)), Northern blotting analysis (Molecular Cloning, Cold Spring Harbor Lab. (1989)), in situ RT-PCR (Nucl. Acids Res., 21, 3159-3166 (1993)), in situ hybridization, Southern blotting (Sambrook, J., et al., Molecular Cloning, a Laboratory Manual. Cold Spring Harbor Laboratory Press: New York. (1989)), fluorescent in situ hybridization (FISH, Takahashi E., et al., Hum. Genet., 86, 1416 (1990)), comparative genomic hybridization (CGH, Kallioneimi, A., et al., Science, 258, 818-821 (1992)), spectral karyotyping (SKY, Rowley, J.D., et al., Blood, 93, 2038-2042 (1999)), and a procedure using the yeast artificial clone (YAC) vector as a probe (Lengauer, C., et al., Cancer Res., 52, 2590-2596 (1992); the NASBA method (nuclei acid sequence-based amplification, Nature, 350, 91-92 (1991)); and various other methods. Detection by RT-PCR is a very suitable procedure.

Detection of MLL4 gene/HBV X gene fusion transcripts by RT-PCR can use a primer set comprising a primer specific for the region containing the MLL4 gene and a primer specific for the HBV X gene.

The length of the RT-PCR primers is the same as in the PCR described previously. The primer specific for the region containing the MLL4 gene and the primer specific for the HBV X gene are also the same as in the PCR described previously.

Specific examples of preferred primer sets for RT-PCR are the set comprising the MD26c shown by SEQ ID NO. 4 as the forward primer and the E5-1 shown by SEQ ID NO. 15 as the reverse primer, and the set comprising the MD24 shown by SEQ ID NO. 13 as the forward primer and the E5-2 shown by SEQ ID NO. 16 or the E6-2 shown by SEQ ID NO. 17 as the reverse primer. Preferred therebetween is the set comprising the MD26c shown by SEQ ID NO. 4 as the forward primer and the E5-1 shown by SEQ ID NO: 15 as the reverse primer.

The amplification product yielded by RT-PCR can be detected by agarose gel electrophoresis, thereby enabling a determination as to HBV X gene integration into the MLL4 gene. The production of an MLL4 gene/HBV X gene fusion transcription product can also be confirmed by determining the base sequence of the fusion product.

Confirmation of the integration of HBV-DNA into MLL4 at the DNA level or transcription product level, as an example indicating the cancerous nature of the tissue, yields a positive finding for the presence of liver cancer.

### Detection of fusion protein

HBV integration into the MLL4 gene can also be detected by detection of MLL4/HBV fusion protein and preferably MLL4/HBV X region fusion protein. The MLL4/HBV X region fusion protein may be a fusion protein between all or a portion of MLL4 and all or a portion of the HBV X region.

This detection can be carried out using an immunoassay that employs antibody that specifically binds to MLL4/HBV X region fusion protein. This antibody also encompasses antibody fragments.

The methods for producing the antibody are well known as such to those skilled in the art, and the present invention can also use these well-known methods (refer, for example, to "Methods in Immunobiochemical Research", Experimental Protocols in Biochemistry Series, edited by The Japanese Biochemical Society (1986)).

The methods known in the subject field can be used without limitation as the immunoassay under consideration and can be exemplified by Western blotting, immunohistological assays, and ELISA assays.

In a specific example of a procedure for detecting fusion protein by ELISA, the target cells presumed to contain MLL4/HBV fusion protein are lysed and the total protein is extracted; incubation is then carried out with monoclonal antibody against fusion protein or HBV X protein; immunoprecipitation is thereafter carried out by an immunoprecipitation procedure; and incubation is subsequently carried out with peroxidase-labeled horse anti-mouse IgG antibody. Proceeding in this manner enables visualization of the signal due to antibody binding. This antigen-binding signal can be detected on electrophoresis gel using an ECL Western Blotting Detection System (Amersham Biosciences).

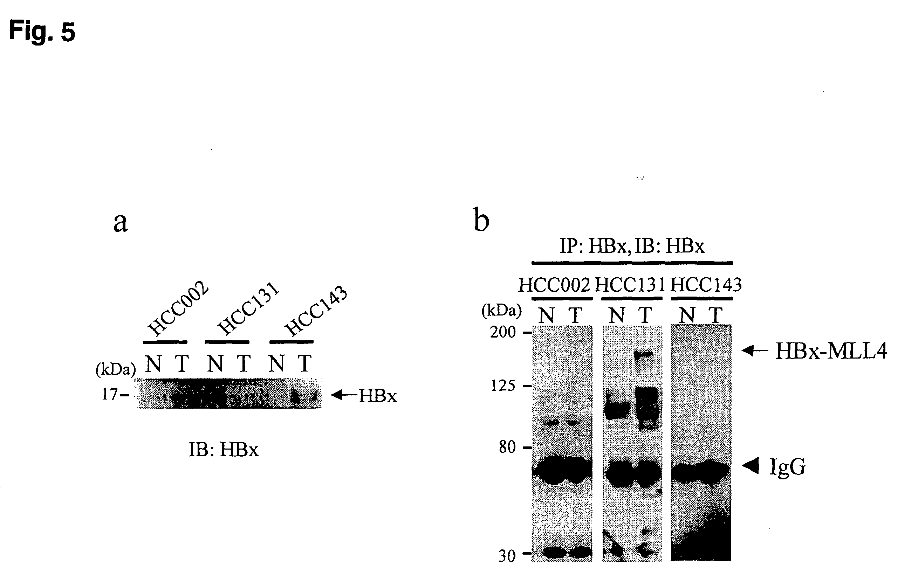

As one example of the detection of MLL4/HBV fusion protein by Western blotting, the detection of MLL4/HBV fusion proteins of various sizes (kDa) in the examples of the present invention is shown in Figure 5.

The detection of fusion protein by the method under consideration and hence the detection of HBV integration yields, as an example indicating the cancerous nature of the tissue, a positive finding for the presence of liver cancer.

### (II) The method of detecting translocation at the MLL4 gene

A second detection method of the present invention is a method that detects, with respect to liver tissue isolated from a human subject and suspected of being cancerous, a t(17;19)(p11.2;q13.1) chromosomal translocation of the MLL4 gene, this translocation indicating the cancerous nature of a tissue.

The human population that is a suitable target for this second method and the procedures for preparing DNA from human samples are the same as in the first method.

### Chromosome 17

The genomic sequence at chromosomal position 17p11.2 on chromosome 17 follows the genomic sequence of the clone with a total base length (bp) of 177,017 reported under GenBank accession number AC087294 by B. Birren et al., of the Whitehead Institute. The base sequence of the region comprising bases 24481 to 25200 of chromosome 17 is shown in SEQ ID NO. 3.

### Detection of translocation by PCR

A specific suitable example of a method that detects a t(17;19) (p11.2;q13.1) chromosomal translocation of the MLL4 gene comprises the execution of PCR using a primer specific to the region containing p11.2 of chromosome 17 and a primer specific to the region containing the MLL4 gene present at q13.1 on the long arm of chromosome 19, followed by detection of the amplification product. The primer specific to the region containing the MLL4 gene is preferably a primer specific to the region containing intron 3 of the MLL4 gene.

Due to the low sensitivity of translocation detection, the primary PCR product is desirably subjected to a secondary PCR using a set comprising a primer specific to the region containing p11.2 of chromosome 17 and a primer specific to the region containing intron 3 of the MLL4 gene present on the long arm of chromosome 19. The primers used in the secondary PCR may in general be designed so as to enable amplification of a region within the region amplified by the primary PCR.

A suitable example of a primer is a nucleotide segment corresponding to DNA designed to be specific for a region of the MLL4 gene in the vicinity of the boundary between chromosome 17 and the MLL4 gene on chromosome 19, having at least 10 and preferably about 15 to about 30 bases in series. A suitable example of the other primer is a nucleotide segment corresponding to DNA designed to be specific for a region of chromosome 17 in the vicinity of the boundary between chromosome 17 and the MLL4 gene on chromosome 19, having at least 10 and preferably about 15 to about 30 bases in series.

As for the first detection method according to the present invention, here "specific" denotes the ability to recognize the indicated region and thereby initiate DNA synthesis.

More specifically, in order to detect translocation from chromosome 19 to chromosome 17, the forward primer for the primary PCR can be exemplified by the primer 671F1 shown by SEQ ID NO. 8, which is an MLL4 gene-specific primer, while the reverse primer can be exemplified by the 17-1 shown by SEQ ID NO. 20, which is a 17q-specific primer. In order to detect translocation from chromosome 17 to chromosome 19, the forward primer for the secondary PCR reaction can be exemplified by the 671F2 shown by SEQ ID NO. 9, which is an MLL4 gene-specific primer, while the reverse primer can be exemplified by the 17-2 shown by SEQ ID NO. 21, which is a 17q-specific primer.

In addition, the forward primer for the primary PCR reaction can be exemplified by the primer 671R1 shown by SEQ ID NO. 10, which is an MLL4 gene-specific primer, while the reverse primer can be exemplified by the 17R1 shown by SEQ ID NO. 18, which is a 17q-specific primer. In order to detect translocation from chromosome 17 to chromosome 19, the forward primer for the secondary PCR reaction can be exemplified by the 671R2 shown by SEQ ID NO. 11, which is an MLL4 gene-specific primer, while the reverse primer can be exemplified by the 17R2 shown by SEQ ID NO. 19, which is a 17q-specific primer.

The detection of the aforementioned translocation by the method according to the present invention, as an example indicating the cancerous nature of the tissue, yields a positive finding for the presence of liver cancer.

The detection method according to the present invention as described in the preceding can detect translocation between chromosome 17 and the MLL4 gene lying on chromosome 19 and, through the comprehension of tissue oncogenesis (for example, hepatocellular carcinoma) in liver tissue suspected of being cancerous and isolated from a human subject suffering from, for example, chronic hepatitis or liver cirrhosis, can thereby provide information and measures useful for the elucidation of the mechanism of onset of hepatocellular carcinoma and for understanding, diagnosing, and preventing oncogenesis in tissues.

### (III) Detection kits

A first detection kit according to the present invention is a kit for detecting integration of HBV-DNA and particularly the X gene of HBV into the MLL4 gene and particularly into its intron 3.

For detection of this integration at the DNA level, the kit can be specifically provided with a primer specific to the region containing intron 3 of the MLL4 gene and a primer specific to the HBV X gene. The primers are as described previously.

A preferred example of the primer set present in this kit is a set comprising a DNA fragment capable of recognizing all or a portion of base sequence shown by SEQ ID NO. 10, 11, or 12 and thereby being capable of initiating DNA synthesis, and a DNA fragment capable of recognizing all or a portion of base sequence shown by SEQ ID NO. 4 and thereby being capable of initiating DNA synthesis. Another preferred example is a set comprising a DNA fragment capable of recognizing all or a portion of base sequence shown by SEQ ID NO. 14 and thereby being capable of initiating DNA synthesis, and a DNA fragment capable of recognizing all or a portion of base sequence shown by SEQ ID NO. 8 or 9 and thereby being capable of initiating DNA synthesis.

The detection kit may contain other components required for PCR. Examples of these other components are labeling agent, Taq DNA polymerase, deoxynucleotide triphosphate, and secondary primer for DNA amplification. The labeling agent can be exemplified by a chemical modifier such as a radioisotope or fluor, and may be conjugated to the DNA fragment itself in advance. The detection kit according to the present invention may also contain, for example, reaction dilution solution, reference antibody, buffer, washing agent, and reaction stop solution suitable for facilitating measurement.

HBV-DNA integration into the MLL4 gene can also be detected using a kit for detecting MLL4 gene/HBV X gene fusion transcription products. In a typical case, a kit for detecting fusion transcription products by RT-PCR can be exemplified by a kit provided with a primer or probe set as described above for detecting integration at the DNA level.

A suitable primer set for this RT-PCR can be exemplified by a set comprising a primer that can recognize base sequence shown by SEQ ID NO. 4 or its complementary base sequence and thereby is capable of initiating DNA synthesis, and a primer that can recognize base sequence shown by SEQ ID NO. 15 or its complementary base sequence and thereby is capable of initiating DNA synthesis.

As has been described above, HBV-DNA integration into the MLL4 gene can also be detected by detecting MLL4/HBV X region fusion protein. The detection kit in this case can be provided with antibody or antibody fragment that specifically recognizes MLL4/HBV X region fusion protein or the X region of HBV. This antibody is the same as that described previously. This kit may also contain essential components in correspondence to the method used, such as, for example, Western blotting, an immunohistological assay, or ELISA.

A second detection kit according to the present invention is a kit for detecting t(17;19) (p11.2;q13.1) chromosomal translocation of the MLL4 gene. This kit is provided with, for example, a primer specific to the region containing intron 3 of the MLL4 gene and a primer specific to the region containing p11.2 of chromosome 17. These primers are the same as described previously.

An example of a suitable primer set is a set for a primary PCR reaction comprising a primer capable of recognizing all or a portion of base sequence shown by SEQ ID NO. 8 or its complementary sequence and thereby being capable of initiating DNA synthesis, and a primer capable of recognizing all or a portion of base sequence shown by SEQ ID NO. 20 or its complementary base sequence and thereby being capable of initiating DNA synthesis. In addition, this kit desirably also contains a primer set for a secondary PCR reaction, wherein the primer set for secondary PCR can be exemplified by a set for detecting translocation from chromosome 17 to chromosome 19, comprising a primer capable of recognizing all or a portion of base sequence shown by SEQ ID NO. 9 or its complementary base sequence and thereby being capable of initiating DNA synthesis, and a primer capable of recognizing all or a portion of base sequence shown by SEQ ID NO. 21 or its complementary base sequence and thereby being capable of initiating DNA synthesis. This kit can also contain the other components necessary for PCR and/or reagents for facilitating measurement.

### EXAMPLES

The present invention is described in additional detail through the examples provided below, but the present invention is not limited to these examples.

### Example 1

### (a) Histological and serological examinations of the

### hepatocyte samples

The following tests were carried out using resected liver tumor tissue (hepatocellular carcinoma or HCC) from 26 Japanese patients obtained at the Second Department of Surgery, Chiba University Hospital, between 1987 and 2003. These patients had not undergone preoperative treatment.

Clinical, histological, and serological examinations were carried out on the patients providing the samples. In the histological examination, the tumor size, degree of differentiation of the tumor cells (three stages), and type of liver disease (CPH: chronic persistent hepatitis; CAH: chronic acute hepatitis; LC: liver cirrhosis) were investigated. In the serological examination, hepatitis B surface antigen (HBsAg) detection was carried out using an enzyme immunoassay (EIA) kit from Dinabot Co., Ltd., and antibody against the hepatitis B surface antigen (anti-HBs antibody) and antibody against the hepatitis B core antigen (anti-HBc antibody) were detected using a radioimmunoassay (RIA) kit, also from Dinabot Co., Ltd. Antibody against the hepatitis C (anti-HCV antibody) was also tested using HCV recombinant antigens c25, c33c, and NS5 as the target antigens and a recombinant immunoblot assay from Ortho Diagnostic Systems.

The results are shown in Table 1 below. In Table 1, HBsAg refers to hepatitis B surface antigen; anti-HBs refers to antibody against hepatitis B surface antigen; anti-HBc refers to antibody against hepatitis B core antigen; anti-HCV refers to antibody against hepatitis C (c25, c33c, and NS5); "mod" refers to moderately differentiated HCC; "poor" refers to poorly differentiated HCC; "well" refers to well differentiated HCC; CPH refers to chronic persistent hepatitis; CAH refers to chronic acute hepatitis; and LC refers to liver cirrhosis. Detection of HBV-DNA was carried out by single PCR using the HBx-specific primers MD24 and MD26.

The use of these samples in the tests conformed to the ethical guidelines of the Declaration of Helsinki (1975) and was also approved by the Ethical Review Board of the School of Medicine of Chiba University.

**Table 1**

| | | | Serological studies | | | | | | | |
|---|---|---|---|---|---|---|---|---|---|---|
| Case no. | Age | Sex | HBsAg | Anti-HBs | Anti-HBc | Anti-HCV | Detection of HBV-DNA¹⁾ | Tumor Size (cm) | Histological findings | Histology of surrounding liver |
| HCC131 | 48 | M | + | - | + | - | positive | 13 | mod | CH |
| HCC146 | 45 | M | + | - | + | - | positive | 3 | poor, mod | LC |
| HCC002 | 58 | M | + | - | + | - | positive | 10 | mod | CH |
| HCC003 | 56 | M | + | - | + | - | positive | 5.5 | mod | LC |
| HCC9907 | 50 | M | + | - | + | - | positive | 2.3 | mod | LC |
| HCC155 | 52 | M | + | - | + | - | positive | | mod | LC |
| H20 | 66 | F | + | - | + | - | positive | 4.7 | mod | CPH |
| H54 | 29 | F | + | - | + | - | positive | 14 | poor | CPH |
| H120 | 41 | M | + | - | + | - | positive | 2.2 | mod | LC |
| HCC143 | 63 | M | + | - | + | + | positive | 4,2 | mod | CPH |
| H49 | 66 | M | - | + | + | + | positive | 3.4 | mod | CAH |
| H62 | 62 | M | - | + | + | + | positive | 2.5 | mod | LC |
| H70 | 53 | M | - | + | + | + | positive | 6 | mod | LC |
| H72 | 62 | M | - | + | + | + | positive | 2.5 | poor | CAH |
| H78 | 66 | M | - | + | + | + | positive | 2 | well | preLC |
| H89 | 61 | F | - | + | + | + | positive | 4 | mod | LC |
| H76 | 71 | M | - | + | + | - | positive | 3.5 | mod | CPH |
| H57 | 66 | M | - | - | + | + | positive | 11 | poor | CAH |
| H71 | 60 | M | - | - | + | + | positive | 1.8 | poor | LC |
| H85 | 55 | M | - | - | + | + | positive | 2.2 | mod | LC |
| H86 | 65 | M | - | - | + | + | positive | 6 | mod | CAH |
| H87 | 62 | M | - | - | + | + | positive | 12 | well | CPH |
| HCC128 | 73 | M | - | - | + | + | negative | 4 | mod | LC |
| HCC147 | 68 | M | - | - | + | + | negative | 2.5 | mod | CPH |
| HCC127 | 57 | M | - | - | + | - | positive | 5 | mod | CPH |
| HCC001 | 68 | M | - | - | + | - | negative | 7 | mod | CH |

### Amplification of HBV integration sites by adaptor-ligation/suppression PCR

### < PCR >

DNAs were extracted from the samples according to a previously reported method (Urashima, T., et al., J. Hepatol., 26, 771-778 (1997)). The following tests were first carried out on 10 samples taken from patients who were HBsAg positive by serological examination. Sequences flanking sites of HBV integration into the human genome (HBV/cellular DNA junctions) in the DNA from these samples were amplified by adaptor-ligation/suppression PCR (Siebert, P.D., et al., Nucleic Acids Res., 23, 1087-1088 (1995)) using a Genomewalker kit from Clontech Laboratories, Inc.

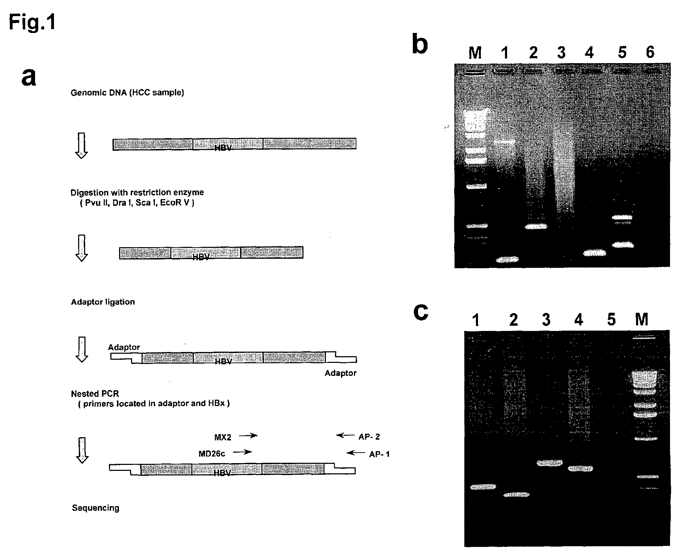

The adaptor-ligation/suppression PCR protocol is described below with reference to Figure 1a. 2.5 µg of genomic DNA was digested with DraI, PvuII, EcoRV, or ScaI restriction enzymes (Life Technologies, Inc.), and adaptor sequences were ligated to the blunt ends thereby produced at both ends of the DNA fragments (ligation). A 50 µL reaction solution was prepared that contained the genomic fragments treated in this manner, 0.2 mM dNTPs, 10 pmol HBV primer with SEQ ID NO. 4 (MD26c), 1X buffer (native Taq buffer, Invitrogen), 4% glycerol, 1 unit Taq (native Taq, Invitrogen), and 0.04 unit Vent DNA polymerase (New England Biolabs) and was heated for 3 minutes at 80°C prior to the PCR reaction. To the resulting solution were then added 2.5 mM MgCl₂ and 10 pmol adaptor-specific primer with SEQ ID NO. 6 (AP-1) followed by reaction for 1 minute at 94°C.

The PCR reaction was then started, but using touchdown PCR as previously reported (Chami, M., et al., Oncogene, 2000; 19: 2877-2866) in order to improve the specificity.

The reaction was run for an initial 20 cycles wherein 1 cycle was 94°C for 30 seconds, 65°C for 30 seconds, and 70°C for 3 minutes and the annealing temperature was dropped 1°C every 2 cycles. The reaction conditions were then held constant at the conditions of cycle 20 (94°C for 30 seconds, 55°C for 30 seconds, and 70°C for 3 minutes) and reaction for another 20 cycles was carried out. The last cycle was followed by reaction for 7 minutes at 70°C, yielding the primary PCR amplification product.

This amplification product was purified using a QIAquick PCR purification kit from QIAGEN Co., Ltd., and, using 1/50 thereof as template, secondary PCR (nested PCR) was carried out using the same procedure, but changing the 10 pmol HBV primer to the MX2 shown by SEQ ID NO. 5 and changing the adaptor-specific primer to the AP-2 shown by SEQ ID NO. 7.

The final amplification product obtained by the preceding procedure was subjected to electrophoresis on 1.2% agarose gel. The results are shown in Figures 1b and 1c.

Each figure shows a typical agarose gel for adaptor-ligation/suppression PCR after the secondary PCR amplification. The PCR products in lanes 1 to 5 of Figure 1b were obtained from PvuII-digested adaptor-ligated DNA libraries. Lanes 1 to 6 show, respectively, HCC9907, HCC002, HCC003, HCC143, HCC146, and the negative PCR control (no DNA). Lane M contains 1 kb DNA size markers (Life Technologies, Inc.).

The PCR conformation using specific primers of HBV-DNA integration into the MLL4 gene is shown in Figure 1c. This figure shows the results of a typical agarose gel from conventional PCR using primers specific for HBx (MD26c: SEQ ID NO. 4) and for MLL4 (19R1: SEQ ID NO. 10 (671R1)).

Lanes 1 to 5 show, respectively, HCC131, HCC143, HCC146, HCC002, and the negative PCR control (no DNA). Lane M contains 1 kb DNA size markers (Life Technologies, Inc.).

### < Determination of the base sequence of the amplified DNA >

Each of the separated bands was then cut out and the base sequence was investigated.

The sequencing reaction was carried out using the generally and widely employed BigDye Terminator Cycle Sequencing kit from Applied Biosystems and was carried out with an ABI PRISM 3700 DNA analyzer. The results are shown in Table 2.

**Table 2.**

| Case no. | Chromosome | Genomic sequence accession number | Gene | t(17;19)(p11.2;q13.1) |
|---|---|---|---|---|
| HCC131 | 19q13.1 | AD000671 | MLL4 | Positive |
| HCC146 | 19q13.1 7p14-15 | AD000671 AC005090 | MLL4 | Positive |
| HCC002 | 19q13.1 | AD000671 | MLL4 | Positive |
| HCC003 | 5p13 | AY007685 | hTERT | Positive |
| HCC9907 | 9q13-21.3 | AL133578 | | Negative |
| HCC155 | | | | Positive |
| H20 | | | | Positive |
| H54 | 18p11.3 | AP000845 | NMPp84^{a)} | Positive |
| H120 | | | | |
| HCC143 | 19q13.1 | AD000671 | MLL4 | Positive |
| H49 | | | | Positive |
| H62 | | | | Negative |
| H70 | | | | Positive |
| H72 | | | | Positive |
| H78 | | | | Positive |
| H89 | | | | Positive |
| H76 | | | | Positive |
| H57 | | | | Negative |
| H71 | | | | Positive |
| H85 | | | | Positive |
| H86 | | | | Negative |
| H87 | | | | Positive |
| HCC128 | | | | Positive |
| HCC147 | | | | Positive |
| HCC127 | | | | Positive |
| HCC001 | | | | Positive |

| | | | | |
|---|---|---|---|---|
| a) H87This integration has already been reported in Oncogene, 20, 62HCC12833-6240 (2001) using HBV-*Alu* PCR. b) "Positive": t(17;HCC14719) (p11.2;q13.1) positive "Negative": t(17;19)(p11.2;HCC127q13.1) negative | | | | |

As shown in Table 2, a total of 8 HBV/cellular DNA junctions were detected among 7 (HCC131, HCC143, HCC146, HCC002, HCC003, HCC9907, H54) of the 10 samples. Among these 8 junctions, 4 (HCC131, HCC143, HCC146, HCC002) were present in intron 3 of the mixed lineage leukemia 4 (MLL4) on 19q13.1. The remainder were present, respectively, in the promoter region of hTERT (human telomerase reverse transcriptase) on 5q13, a region encoding a hypothetical protein on 9q13-21.3, and an expressed sequence tag (EST) region on 7p14-15. Two groups have already reported the integration of HBV into the hTERT region (Ferber, M.J., et al., Oncogene, 22, 3813-3820 (2003); Paterlini-Brechot, P., et al., Oncogene, 22, 3911-3916 (2003)), and these reports are supported by the results reported herein.

### (c) Amplification of the HBV integration site by PCR using primers specific to the MLL4 gene

Based on the preceding results, integration of HBV-DNA into the MLL4 gene was confirmed (Figure 1c) by carrying out conventional PCR on the 4 genomic DNAs in which an HBV/MLL4 junction was observed, using a combination of the HBV-specific primer MD26c (SEQ ID NO. 4) with the MLL4-specific primer 671R1 (SEQ ID NO. 10), 671R2 (SEQ ID NO. 11), or 671R3 (SEQ ID NO. 12). The other MLL4/HBV junction was confirmed by PCR using a combination of the HBV-specific primer MD26 shown by SEQ ID NO. 14, which is complementary to MD26c, with the MLL4-specific primer 671F1 (SEQ ID NO. 8) or 671F2 (SEQ ID NO. 9).

The details of the test procedure are as follows. A 50 µL reaction solution was prepared that contained 100 ng of genomic DNA, 0.2 mM dNTPs, 10 pmol of each primer, 1XGCI buffer (Takara), and 1 unit LA Taq DNA polymerase (Takara). The reaction for investigating MLL4/HBV junctions used the primers 671F1 and MD26, and consisted of reaction for 35 cycles of heating for 1 minute at 94°C and then 10 seconds at 98°C and 1 to 4 minutes at 68°C. An extension reaction for 10 minutes at 72°C was finally carried out. The preceding constituted the primary PCR.

Secondary PCR was then carried out under the same conditions as before using the primers 671F2 and MD26. The reaction for investigating HBV/MLL4 junctions used the primers MD26c and 671R3, and consisted of reaction for 30 cycles of heating for 1 minute at 94°C and then for 30 seconds at 94°C, 30 seconds at 50°C, and 45 seconds at 72°C, followed finally by an extension reaction for 5 minutes at 72°C.

The results are shown in Figures 2 and 3. Figure 2 shows the sequences flanking the HBV integration sites in DNAs amplified by the PCR described above.

The HBV/cellular DNA junction sequences in the MLL4 gene for HCC131, HCC143, HCC146; and HCC002 of Table 1 are shown in Figure 2a. In sequences 1) to 4), the sequences to the left of the slash show the sequence of the integrated HBV-DNA, while the sequence shown on the right in bold is the flanking MLL4 gene sequence. The numbers appearing above sequences 1) to 4) designate the HBV nucleotide number (GenBank accession number AB033550, HBV genotype C, subtype adr)) at the HBV/cellular DNA junction.

The sequence of intron 3 of the MLL4 gene (GenBank accession number AD000671) and the sequences flanking the HBV-DNA integration sites are shown in Figure 2b.

The results of the analysis of the HBV genome integrated into the DNA of the 4 samples from HCC002, HCC131, HCC143, and HCC146 are shown in Figure 3. A cartoon of the gene structure of the HBV genome (GenBank accession number AB033550) is shown in Figure 3a, wherein the open reading frame (ORF) and the direction of transcription are shown by the thick arrows, and the numbers thereabove show the positions of the open reading frames (ORF). DR1 and DR2 are 11-base pair direct repeats.

A cartoon of the HBV genomes integrated into the DNA of the 4 samples from HCC002, HCC131, HCC143, and HCC146 is given in Figure 3b, wherein the solid circle and the arrow indicate the 5' terminal and 3' terminal of the integrated HBV-DNA sequence. The 5' terminal of the HBV-DNA sequence integrated in HCC146 could not be determined.

A cartoon of intron 3 (corresponds to nucleotides 17316 to 17869 of GenBank accession number AD000671) of the MLL4 gene and the HBV-DNA at the integration site is given in Figure 3c, which includes cartoons of the orientation and position of the MLL4-specific primers used in this analysis.

As is shown in Figures 2 and 3, it was confirmed that, in these 4 genomic DNAs, HBV integrated in or adjacent to an Alu repeat sequence present in intron 3 of MLL4.

In general, an integrating region of HBV frequently occurs in the vicinity of the DR1 and DR2 containing 11-base pair repeat sequences, and in the 4 examples under consideration here integration occurred in all cases in the vicinity of DR1. Integration of full-length HBV was confirmed only for HCC131, while partial integration was observed in the other 3 cases (Figure 3). HBV infections in the Japanese are more commonly cases exhibiting genotype C as in HCC131.

In addition, the presence of HCV-DNA was confirmed in all of the HCC samples (Table 1) by running PCR that combined the HBx-specific primer MD24 shown by SEQ ID NO. 13 with the HBx-specific primer MD26 shown by SEQ ID NO. 14.

### Example 2

### Detection of HBx/MLL4 fusion transcription products by RT-PCR

The coding region for HBx protein excluding the C-terminal and the promoter region of HBx (X gene of HBV-DNA) were present in all 4 cases in which MLL4/HBV-DNA integration had been confirmed. The presence of HBx/MLL4 fusion transcription products in these 4 samples was predicted on this basis. The total RNA was extracted from HCC131, HCC143, HCC146, and HCC002 and the transcription products obtained by RT-PCR were investigated.

The details of the test procedure are given below.

The RNA was extracted using Trizol (Invitrogen) according to the instructions for use. The extracted RNA was confirmed by electrophoresis on 1.2% agarose gel. RT-PCR was carried out as follows using a Superscript One-Step RT-PCR system (Invitrogen) according to the instructions for use; gene-specific primers designed on exon 5 or 6 of MLL4 (E5-1: SEQ ID NO. 15; E5-2: SEQ ID NO. 16; and E6-1: SEQ ID NO. 17) were used as the reverse transcription primers. 500 ng of the individual RNA, 0.8 µM of each primer (MD26c with SEQ ID NO. 4 was used as the common sense primer), 2 units of an enzyme mixture (contained SuperScript II RT and Platinum Taq DNA polymerase), and 1X buffer (contained 0.2 mM dNTPs and 1.2 mM MgSO₄) were mixed on ice to prepare the reaction solution. This reaction solution was reacted for 30 minutes at 50°C and 2 minutes at 94°C with thorough stirring with a vortex. The ensuing PCR reaction consisted of reaction for 30 cycles of 15 seconds at 94°C, 30 seconds at 55°C, and one minute at 70°C. The PCR product was subjected to electrophoresis on 1.2% agarose gel, and each of the resulting bands was cut out and subjected to sequence analysis.

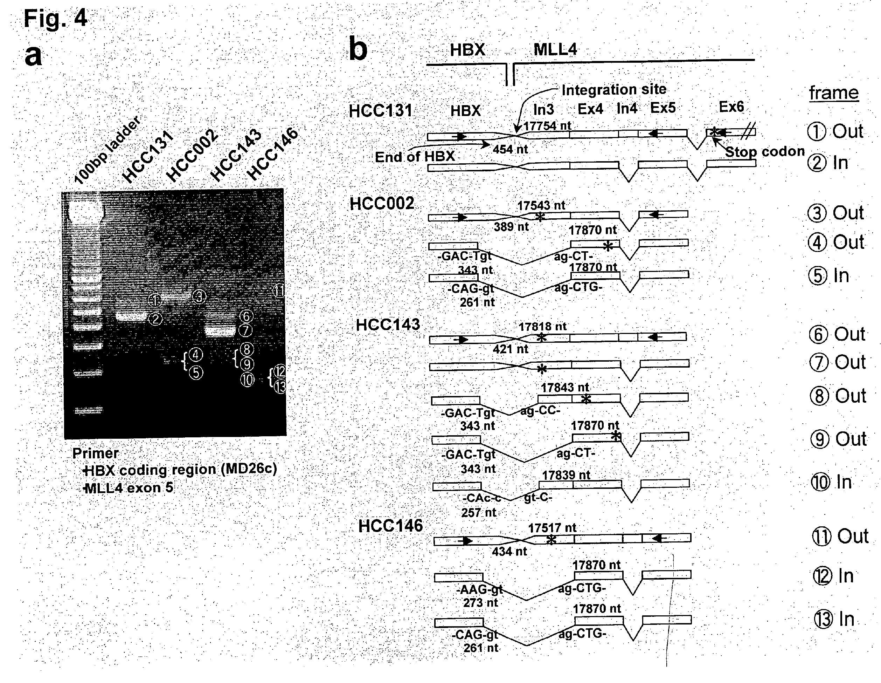

The results of RT-PCR analysis for HBx-MLL4 fusion transcripts are shown in Figure 4.

As shown in Figure 4a, various fusion transcripts were obtained from each hepatocellular carcinoma tissue by RT-PCR using MD26c/MLL exon 5 primers. The RT-PCR products in the individual bands in Figure 4a were subjected to base sequence analysis. A cartoon of the fusion transcription products from the 4 hepatocellular carcinoma tissues (HCC131, HCC002, HCC143, and HCC146) is given in Figure 4b. The numbers in the circles displayed vertically at the individual bands in Figure 4a correspond to the numbers in the vertically displayed circles for the fusion transcription products in Figure 4b.

As a result, in-frame fusion transcription products from exon 4 onward of MLL4 and out-of-frame fusion transcription products containing a premature stop codon were detected in all the hepatocellular carcinomas (Figure 4b).

Two fusion transcription products were detected in HCC131 (an in-frame fusion transcription product that included an intron 3 sequence and the sequence from exon 4 onward, and a fusion transcription product that included intron 3 and intron 4 and that had a premature stop codon in exon 6) and three fusion transcription products were detected in HCC002 (an in-frame fusion transcription product from which intron 3 had been spliced out; a fusion transcription product from which intron 3 had been similarly spliced out, but which was out-of-frame and had a premature stop codon in exon 4; and a fusion transcription product that included intron 3 and had a premature stop codon in intron 3). Three fusion transcription products with a pattern resembling HCC002 were detected in HCC146. A total of 4 species of out-of-frame fusion transcription products were detected in HCC143, 2 species in which stop codons were present in intron 3 and 2 species in which stop codons were present in exon 4. One species of in-frame fusion transcription product that included intron 3 was also detected in HCC143; splicing occurred at a CC-GT site in this fusion product, rather than by the GT-AG rule.

### Example 3

### Detection of HBx/MLL4 fusion proteins by immunoblotting analysis

In order to confirm the actual translation of these fusion transcription products into protein, a comparison was carried out by immunological test procedures using liver tumor cells and cells adjacent thereto for HCC131, HCC143, and HCC002.

The details of the test procedures are as follows. The complete protein was prepared by lysing 100 to 200 mg of cells with a buffer that contained 0.1% SDS, 0.5% deoxycholate, 1% NP-40, 150 mM NaCl, 50 mM Tris-HCl (pH 8.0), 1 mM APMSF, and other protease inhibitors (complete protease inhibitor tablets, Roche) followed by centrifugation at 12,500 rpm for 30 minutes at 4°C. Optionally, 500 µg of the obtained total protein was incubated with anti-HBx (hepatitis B virus X protein) monoclonal antibody (Advanced Immuno Chemical, Inc.), and immunoprecipitation was performed by a standard method.

The total protein and the immunoprecipitated product prepared by the preceding procedure were electrophoresed by SDS-PAGE and transferred to a PVDF membrane (Hybond-P, Amersham Biosciences). The membrane was pre-incubated for 1 hour at room temperature with TBS-T (Tris-buffered saline and 0.1% Tween-20) containing 3% skim milk in order to prevent nonspecific antibody binding and was then incubated for 8 hours at 4°C with anti-HBx monoclonal antibody. This was followed by washing 4 times with TBS-T and then incubation with peroxidase-conjugated horse anti-mouse IgG. The signal due to antibody binding was visualized with an ECL Western blotting detection system (Amersham Biosciences) and was analyzed.

The results are shown in Figure 5. Western blot analysis using anti-HBx monoclonal antibody is given in Figure 5a for tumor tissue (T) and adjacent nontumor tissue (N) from HCC002, HCC131, and HCC143, while Figure 5b shows the immunoprecipitation product due to immunodetection with anti-HBx antibody.

According to the results of direct Western blotting on the total protein, a 17 kDa protein was selectively detected in the tumor cells of HCC143 and HCC002. This corresponds to a short HBx/MLL4 fusion protein due to the presence of a premature stop codon (Figure 5a). While fusion protein was not detected with HCC131, a 160 kDa protein corresponding to the in-frame HBx/MLL4 fusion protein was detected in the Western blot against the immunoprecipitation product using anti-HBx antibody (Figure 5b).

### Example 4

### Amplification of chromosomal translocation regions by touchdown PCR

The integration of HBV into intron 3 of MLL4 was investigated for subjects who were HBs antigen negative but anti-HBc positive (see Table 1). A primary PCR was run using the HBV primer MD26c (SEQ ID NO. 4) and the MLL4-specific primer 19R1 (SEQ ID NO. 10 (671R1)) and a secondary PCR was run using the HBV primer MD26c (SEQ ID NO. 4) and the MLL4-specific primer 19R2 (SEQ ID NO. 11 (671R2)), followed by sequence analysis according to the method of Example 1. HBV integration into intron 3 of MLL4 was not detected in these hepatocellular carcinoma (HCC) samples as a result.

However, chimeric sequences between MLL4 and the 17p11.2 region were detected instead. Since this translocation was expressed by accident in the tests using HBV-specific primer and MLL4-specific primer, primers 17R1 (SEQ ID NO. 18) and 17R2 (SEQ ID NO. 19) specific for 17p were designed to replace the HBV-specific primer. Using these primers, confirmation of translocation between chromosomes 17 and 19 was carried out by the previously described touchdown PCR on the 26 samples from hepatocellular carcinoma patients.

In order to detect the chromosome 17/chromosome 19 boundary, primers 19R1 and 17R1 were used in a primary PCR and primers 19R2 and 17R2 were used in a secondary PCR. In order to detect the chromosome 19/chromosome 17 boundary, primary PCR was run using primers 671F1 (SEQ ID NO. 8) and 17-1 (SEQ ID NO. 20) and secondary PCR was run using primers 671F2 (SEQ ID NO. 9) and 17-2 (SEQ ID NO. 21).

This resulted in the confirmation of translocation in 22 of 26 samples from hepatocellular carcinoma patients (refer to Table 2).

A sequence diagram showing reciprocal translocation in the gene locus of intron 3 of MLL4 is shown in Figure 6. The rearrangement of chromosome 19q13.1 (left, GenBank accession number AD000671) and chromosome 17p11.2 (right, GenBank accession number AC087294) is shown in Figure 6. An Alu repeat sequence is shown by the sequence within the gray frame. The HBV integration sites lying in and adjacent to the Alu repeat sequence are shown by arrows, and the downward-pointing thick arrows for each chromosome show the newly synthesized chromosomes after the generation of recombination by the Alu repeat sequence (black from black and white from white).

The results of the sequence analysis, shown in Figure 6, demonstrated that a region stretching over about 240 bp and forming the chromosomal boundary in the translocation products, contains an Alu repeat sequence and is about 85% homologous between the chromosomes.

Although it is thought possible that homologous recombination mediated by the Alu sequences could promote translocation, the same translocation was also observed in HCC003, where HBV was integrated into the promoter region of hTERT and not in the intron 3 region of MLL4. Thus, it is also possible that oncogenesis in liver cancer itself causes genomic instability. In addition, in the analysis of this translocation, uncertain results were frequently obtained at the primary PCR stage, making it desirable to carry out a secondary PCR.

In addition, a CA repeat sequence is present adjacent to the Alu sequence on chromosome 17, which will be effective, for example, for individual identification.

## Claims

1. A method for detecting, in liver tissue isolated from a human subject and suspected of being cancerous, integration of HBV-DNA into the MLL4 gene, said integration of HBV-DNA indicating cancerous nature of a tissue.

2. The method according to claim 1, for detecting integration of HBV-DNA into the MLL4 gene by
conducting a polymerase chain reaction (PCR) on DNA from the liver tissue; and
confirming the integration of HBV-DNA into the MLL4 gene by determining the base sequence of the amplified DNA.

3. The method according to claim 2, wherein the integration of a region containing the HBV-DNA X gene into intron 3 of the MLL4 gene is confirmed.

4. The method according to claim 1, for detecting the integration of HBV-DNA into intron 3 of the MLL4 gene.

5. The method according to claim 4, for detecting the integration of HBV-DNA into the region of intron 3 of the MLL4 gene from base number 17515 to 17818 from the 5' end.

6. The method according to claim 1, 4, or 5, wherein the HBV-DNA is a region containing the X gene of HBV.

7. A method for detecting, in liver tissue isolated from a human subject and suspected of being cancerous, integration of HBV-DNA into the MLL4 gene, said integration of HBV-DNA indicating the cancerous nature of a tissue, by a procedure comprising the steps of:
(1) extracting DNA from the liver tissue;
(2) carrying out PCR using, with the DNA obtained in (1) being used as template, a first primer specific to the region containing intron 3 of the MLL4 gene and a first primer specific to the X gene region of HBV; and
(3) optionally carrying out PCR again using ,with the DNA amplified in (2) being used as template, a second primer specific to the region containing intron 3 of the MLL4 gene and a second primer specific to the X gene region of HBV.

8. The method according to any of claims 1, 4, 5, and 6, wherein integration of HBV-DNA into the MLL4 gene is detected by detecting an MLL4 gene/HBV fusion transcription product.

9. The method according to claim 8, for detecting an MLL4 gene/HBV X region fusion transcription product.

10. The method according to any of claims 1, 4, 5, and 6, wherein integration of HBV-DNA into the MLL4 gene is detected by detecting an MLL4/HBV fusion protein.

11. The method according to claim 10, comprising detecting an MLL4/HBV X region fusion protein.

12. The method according to claim 10 or 11, which uses an antibody or antibody fragment that specifically binds to an MLL4/HBV X region fusion protein.

13. A method for detecting, in liver tissue isolated from a human subject and suspected of being cancerous, a t(17;19) (p11.2;q13.1) chromosomal translocation of the MLL4 gene, said translocation indicating the cancerous nature of a tissue.

14. The method according to claim 13, for detecting the t(17;19) (p11.2;q13.1) chromosomal translocation of the MLL4 gene by detecting a base sequence that includes a junction between chromosome 17 and chromosome 19.

15. The method according to claim 14, for detecting a base sequence containing a junction between chromosome 17 and chromosome 19 by a procedure comprising the steps of:
(1) extracting DNA from the liver tissue;
(2) carrying out PCR using, with the obtained DNA being used as template, a first primer specific to a region containing p11.2 of chromosome 17 and a first primer specific to a region containing intron 3 of the MLL4 gene at q13.1 of chromosome 19; and
(3) carrying out PCR using, with the amplified DNA being used as template, a second primer specific to the region containing p11.2 of chromosome 17 and a second primer specific to the region containing intron 3 of the MLL4 gene at q13.1 of chromosome 19.

16. A kit for detecting the integration of HBV-DNA into the MLL4 gene.

17. A kit for detecting the integration of HBV-DNA into intron 3 of the MLL4 gene.

18. A kit for detecting the integration of the X region of HBV into intron 3 of the MLL4 gene.

19. The kit according to claim 18, comprising a primer or probe specific to the region containing intron 3 of the MLL4 gene and a primer or probe specific to the X region of HBV.

20. A kit for detecting an MLL4 gene/HBV X region fusion transcription product.

21. A kit for detecting an MLL4 gene/HBV X region fusion protein.

22. The kit according to claim 21, comprising an antibody or antibody fragment that specifically binds to an MLL4/HBV X region fusion protein.

23. A kit for detecting a t(17;19) (p11.2;q13.1) chromosomal translocation of the MLL4 gene.

24. The kit according to claim 23, comprising a primer or probe specific to the region containing intron 3 of the MLL4 gene and a primer or probe specific to the region containing p11.2 of chromosome 17.
